# EUROPEAN PATENT APPLICATION

(11) **EP 4 799 710 A1**
(43) Date of publication of application: **02.09.2026**
(21) Application number: 24882781.8
(22) Date of filing: 22.10.2024
(51) Int. Cl.: B01D 3/26, C07D 207/267, C07B 63/00

(54) **SLUDGE SEPARATOR, AND NMP PURIFICATION METHOD AND APPARATUS USING SAME**

(30) Priority: 25.10.2023 KR 20230143732
(71) Applicant: Duksan Co., Ltd., Anseong-si, Gyeonggi-do 17601 (KR); Hanmo Corporation, Seoul 08390 (KR)
(72) Inventor: LEE, Seungyong, Hongseong-gun, Chungcheongnam-do 32210 (KR); LEE, Wooyong, Yongin-si, Gyeonggi-do 16990 (KR); LEE, Heejae, Bucheon-si, Gyeonggi-do 14581 (KR); YOO, Insung, Seoul 08848 (KR)
(74) Representative: Ex Materia
(86) International application number: PCT/KR2024/016100
(87) International publication number: WO 2025/089754

(57) **Abstract**

A sludge separator according to the present invention comprises a barrier layer having a plurality of barrier units spaced apart from each other, and thus the sludge separator has excellent performance and can prevent a gas flow path from being blocked. The NMP purification method and apparatus using the sludge separator can provide high-purity NMP by improving the recovery rate of NMP recovered from waste NMP.

## Description

### [Technical Field]

The present invention relates to a sludge separator, and NMP purification method and apparatus using the same.

### [Background Art]

Recently, as technology development for portable devices such as mobile phones and cameras has increased, interest in secondary batteries, which are energy sources, is increasing. Among such secondary batteries, as the demand for lithium secondary batteries that exhibit high energy density and operating potential, have a long cycle lifespan, and have a low self-discharge rate rapidly increases, research to recycle them is also continuing.

N-methyl-2-pyrrolidone (NMP), which is an organic solvent, has chemical stability, low volatility, and the property of being able to dissolve various substances, so it is widely used as a solvent in industries such as petrochemical, plastic, and pharmaceutical industries, and is also used in the process of manufacturing secondary batteries.

In particular, a lithium ion secondary battery is composed of a positive electrode manufactured by coating an electrode material including a lithium compound such as lithium cobaltate and lithium manganate, a binder such as polyvinylidene fluoride, and N-methyl-2-pyrrolidone (NMP) as a solvent on a substrate and then sintering it, and a negative electrode manufactured by coating an electrode material including a compound including carbon, titanium, etc., a binder such as polyvinylidene fluoride, and water as a solvent on a substrate and then sintering it. As such, NMP is not only used as a main material for manufacturing a positive electrode in an electrode manufacturing process, but is also discharged in a large amount as it is used for cleaning and drying, and thus research on technology to effectively recover it is continuing.

For example, Korean registered Patent No. 2050990 discloses a system for separating and recovering NMP from NMP gas by bringing NMP-containing gas containing NMP into gas-liquid contact with water that has absorbed NMP, whereby the NMP of the NMP-containing gas is absorbed by the water.

As for NMP used in secondary batteries, the larger the amount of product recovered from a commercial recycling apparatus, the lower the manufacturing cost can be. However, in case of NMP waste liquid for secondary batteries where a large amount of sludge is generated, if the large quantity of sludge is not treated smoothly, the sludge is introduced into the supply stage of the purification tower, causing a problem where the purification column becomes blocked. In addition, to treat this sludge, it must be discharged to the exterior using a transfer liquid mainly containing an NMP solution, but in this case, the recovery rate of NMP is lowered.

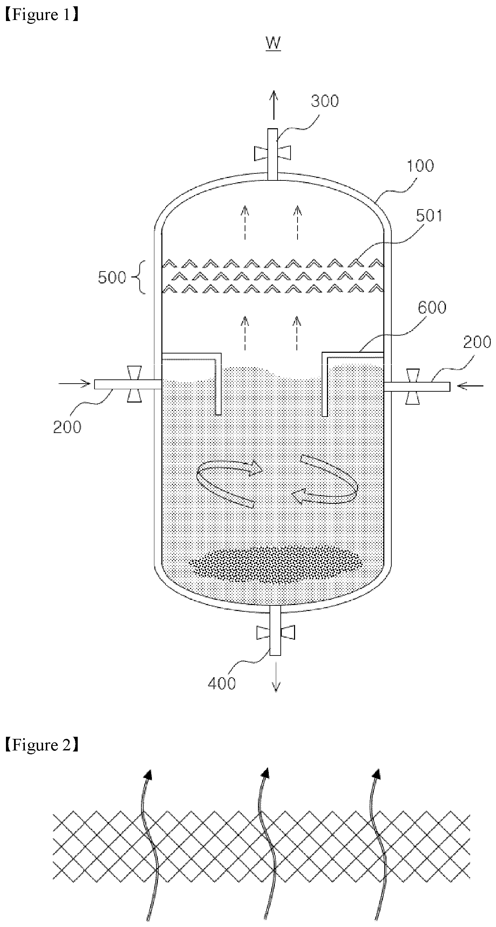

To solve this problem, a sludge separator equipped with a filter of a mesh net as shown in FIG. 2 is used to prevent the sludge inflow. However, if sludge with viscosity adheres to this mesh net, the flow path becomes blocked, and there is a problem that evaporation gas cannot pass through the filter.

### [Prior Art Document]

(Patent Document 1) Korean registered Patent No. 2050990

### [Disclosure]

### [Technical Problem]

As a result of research by the present inventors, by arranging a specially designed barrier unit inside a sludge separator, it was possible to embodiment a sludge separator that can solve the problems of a conventional mesh net filter.

Therefore, an object of the present invention is to provide a sludge separator that has excellent sludge separation performance and does not cause blockage of a gas flow path. In addition, an object of the present invention is to provide a purification method and a purification apparatus for NMP that can obtain high purity NMP from waste NMP with a high recovery rate by using the sludge separator.

### [Technical Solution]

The present invention provides a sludge separator, comprising: a container (100); an inlet (200) through which waste liquid is supplied to the container; a gas outlet (300) formed at an upper end of the container; a liquid phase outlet (400) formed at a lower end of the container; and a sludge barrier (500) disposed on an upper side of the container interior and having a plurality of barrier layers; wherein the barrier layer includes a plurality of barrier units (501) having a cross-section of a conical hat shape and spaced apart from each other.

In addition, the present invention provides an NMP purification method, comprising: (1) a step of introducing waste NMP from a raw material tank into the above-described sludge separator; (2) a step of discharging a first gas mixture including NMP separated in the sludge separator through an upper end of the sludge separator, and discharging a mixture including sludge through a lower end of the sludge separator; (3) a step of performing a first purification by introducing the discharged first gas mixture including NMP into a first distillation tower; (4) a step of discharging an effluent including water separated through the first purification to outside the system through an upper end of the first distillation tower, and discharging first bottoms through a lower end of the first distillation tower; (5) a step of performing a second purification by introducing the discharged first bottoms into a second distillation tower; (6) a step of discharging purified NMP separated through the second purification to outside the system through an upper end of the second distillation tower, and discharging second bottoms through a lower end of the second distillation tower; (7) a step of performing a heat treatment on the mixture including sludge discharged in step (2) by introducing it into a filtrate recovery apparatus; (8) a step of discharging third bottoms separated through the heat treatment to outside the system through a lower end of the filtrate recovery apparatus, and discharging a second gas mixture including NMP through an upper end of the filtrate recovery apparatus; (9) a step of introducing the second bottoms discharged in step (6) and the second gas mixture including NMP discharged in step (8) into an impurity tank to be mixed; and (10) a step of circulating the mixture from the impurity tank to the raw material tank.

Furthermore, the present invention provides an NMP purification apparatus, comprising: a raw material tank for supplying waste NMP; a sludge separator having the above-described constitution, which discharges a first gas mixture including NMP separated from the supplied waste NMP through an upper end and discharges a mixture including sludge through a lower end; a first distillation tower which performs a first purification on the first gas mixture including NMP, discharges an effluent including water separated through the first purification to outside the system through an upper end, and discharges first bottoms through a lower end; a second distillation tower which performs a second purification by distilling the first bottoms, discharges purified NMP separated through the second purification to outside the system through an upper end, and discharges second bottoms through a lower end; a filtrate recovery apparatus which performs a heat treatment on the mixture including sludge discharged from the sludge separator, discharges third bottoms separated through the heat treatment to outside the system through a lower end, and discharges a second gas mixture including NMP through an upper end; and an impurity tank which mixes the second bottoms discharged from the second distillation tower and the second gas mixture including NMP discharged from the filtrate recovery apparatus and introduces the mixture into the raw material tank.

### [Advantageous Effects]

A sludge separator according to the present invention can have excellent sludge separation performance while preventing blockage of a gas flow path. Specifically, according to the present invention, it is possible to solve the problems of a conventional mesh net filter by arranging a specially designed barrier unit within the sludge separator.

Therefore, the purification method and apparatus for NMP using the sludge separator of the present invention can improve the recovery rate of NMP recovered from waste NMP, thereby providing NMP of high purity.

### [Description of Drawings]

FIG. 1 is a cross-sectional view of a sludge separator according to an embodiment of the present invention.
FIG. 2 illustrates the flow of gas in a mesh net filter according to the prior art.
FIG. 3 illustrates the flow of gas in a sludge barrier according to an embodiment of the present invention.
FIG. 4 is a cross-sectional view of a sludge barrier according to an embodiment of the present invention.
FIG. 5 is an exploded perspective view of a sludge barrier according to an embodiment of the present invention.
FIG. 6 is a perspective view of a sludge barrier according to an embodiment of the present invention.
FIG. 7 illustrates a fastening structure of a barrier unit and a connection beam according to an embodiment.
FIG. 8 is a plan view of a sludge barrier according to an embodiment of the present invention.
FIG. 9 illustrates a constitution of an NMP purification apparatus according to an embodiment of the present invention.

### <Description of Reference Numerals>

W: sludge separator, 100: container, 200: inlet, 300: gas outlet, 400: liquid phase outlet, 500: sludge barrier, 501: barrier unit, 505: connection beam, 509: fastening means, 510: first barrier layer, 520: second barrier layer, 530: third barrier layer, 600: separation plate, a: angle of conical hat, L: length of one side of conical hat, dL: vertical spacing between barrier layers, dU: horizontal spacing between barrier units, A: vacuum pump, B1: preheater, B2: sludge separator heater, B3: first reboiler, B4: second reboiler, D1, D2: first and second distillation towers, P1 to P6: first to sixth pumps, R: filtrate recovery apparatus, RT: raw material tank, PT: impurity tank, ST: storage tank, T-1 to T-5, Y-1 to Y-4: discharge pipes, S-1 to S-5: circulation pipes, X-1 to X-5: input pipes.

### [Best Mode for Carrying Out the Invention]

Hereinafter, the present invention will be described in detail with reference to the drawings.

In this specification, when it is determined that a detailed description of a related known constitution or function may obscure the essence of the present invention, the detailed description thereof will be omitted. In addition, the size of each component in the drawings may be exaggerated or omitted for the convenience of description, and may be different from the actually applied size.

In this specification, when a certain part is said to "include" a certain component, it means that it may further include other components rather than excluding other components, unless there is a particularly contradictory description.

All numbers and expressions indicating the amount of components, reaction conditions, etc., described in this specification should be understood as being modified by the term "about" in all cases, unless there is a special description.

In the following description, a description that one component is formed above/under another component or is connected or coupled to each other includes all cases of being indirectly formed, connected, or coupled via another component or directly between these components.

In this specification, terms such as primary, secondary, first, second, etc., are used to describe various components, and the components are not limited by the terms. The terms are used only for the purpose of distinguishing one component from another component.

In this specification, when a numerical range with a defined upper limit and a numerical range with a defined lower limit are described to exemplify the size, physical properties, etc., of a component, it should be understood that a numerical range combining these upper limit and lower limit is also included in the exemplary range of the present invention.

In this specification, a singular form expression should be interpreted as having a meaning that includes the singular or plurality as interpreted in the context, unless there is a special description.

The present invention is not limited to the content disclosed below, but may be modified into various forms as long as the essence of the invention is not changed.

### Sludge Separator

FIG. 1 shows a cross-sectional view of a sludge separator according to an embodiment of the present invention.

Referring to FIG. 1, a sludge separator (W) according to an embodiment includes a container (100); an inlet (200) through which waste liquid is supplied to the container; a gas outlet (300) formed at an upper end of the container; a liquid phase outlet (400) formed at a lower end of the container; and a sludge barrier (500) disposed on an upper side of the container interior and having a plurality of barrier layers, wherein the barrier layer includes a plurality of barrier units (501) having a cross-section in the shape of conical hat and spaced apart from each other.

In the sludge separator (W) according to the embodiment, the waste liquid is supplied to the interior of the container (100) through the inlet (200), and the sludge contained in the waste liquid precipitates to a lower part of the container. The gas evaporated from the waste liquid moves to an upper part of the container and passes through a gap between the barrier units (501) of the sludge barrier (500), but fine sludge contained in the gas is blocked by the barrier units (501) and falls down. Thereafter, the gas from which the sludge is blocked is discharged to the exterior through the gas outlet (300) at the upper end of the container (100), and the sludge precipitated in the lower part of the container (100) is discharged to the exterior through the liquid phase outlet (400).

Hereinafter, each component of the sludge separator will be described in detail.

The shape of the container (100) may be, for example, cylindrical as illustrated in FIG. 1.

The diameter in a horizontal cross-section of the container (100) may be, for example, 500 mm or more, 800 mm or more, or 1,000 mm or more, and may also be 3,000 mm or less, 2,500 mm or less, or 2,000 mm or less. Specifically, the diameter in the horizontal cross-section of the container (100) may be 500 mm to 3,000 mm. More specifically, the diameter in the horizontal cross-section of the container (100) may be 800 mm to 2,500 mm.

The height in a vertical cross-section of the container (100) may be, for example, 1,000 mm or more, 1,200 mm or more, or 1,600 mm or more, and may also be 4,000 mm or less, 3,000 mm or less, or 2,500 mm or less. Specifically, the height in the vertical cross-section of the container (100) may be 1,000 mm to 4,000 mm. More specifically, the height in the vertical cross-section of the container (100) may be 1,200 mm to 2,500 mm.

The wall thickness of the container (100) may be, for example, 5 mm to 10 mm. More specifically, the wall thickness of the container (100) may be, for example, 5 mm to 8 mm.

A material of the wall surface of the container may be, for example, stainless steel (e.g., STS304, STS316(L)), Hastelloy alloy, or the like.

Referring to FIG. 1, the inlet (200) through which waste liquid is supplied is formed in the container (100). For example, the inlet may be formed on a side surface of the container. Through the inlet, waste liquid containing sludge is supplied to the sludge separator.

The diameter of the inlet (200) may be, for example, 15 mm or more, 30 mm or more, or 45 mm or more, and may also be 100 mm or less, 80 mm or less, or 60 mm or less. Specifically, the diameter of the inlet (200) may be 15 mm to 100 mm. More specifically, the diameter of the inlet may be 15 mm to 80 mm.

In addition, the angle of the inlet may be appropriately adjusted so that the waste liquid flows into the container interior in an oblique direction to form a cyclone rotation.

A pipe for supplying the waste liquid may be connected to the inlet. Furthermore, a valve for adjusting a supply amount of the waste liquid may be installed in the inlet or the pipe connected thereto.

Referring to FIG. 1, the gas outlet (300) is formed at an upper end of the container (100). Through the gas outlet, gas generated through evaporation from the waste liquid supplied to the container interior is discharged to the exterior.

The diameter of the gas outlet (300) may be, for example, 150 mm or more, 200 mm or more, or 250 mm or more, and may also be 500 mm or less, 400 mm or less, or 300 mm or less. Specifically, the diameter of the gas outlet (300) may be from 150 mm to 500 mm. More specifically, the diameter of the gas outlet may be from 200 mm to 400 mm.

A pipe for transferring the gas to a subsequent process such as additional purification may be connected to the gas outlet. In addition, a valve for adjusting the discharge amount of the gas may be installed in the gas outlet or the pipe connected thereto.

Referring to FIG. 1, the liquid phase outlet (400) is formed at a lower end of the container (100). Through the liquid phase outlet, the waste liquid containing sludge sedimented in the lower part of the container is discharged.

The diameter of the liquid phase outlet (400) may be, for example, 40 mm or more, 80 mm or more, or 120 mm or more, and may also be 400 mm or less, 350 mm or less, or 300 mm or less. Specifically, the diameter of the liquid phase outlet (400) may be from 40 mm to 400 mm. More specifically, the diameter of the liquid phase outlet may be from 80 mm to 350 mm.

A pipe for transferring the sludge solution to a subsequent process such as filtrate recovery may be connected to the liquid phase outlet. In addition, a valve for adjusting the discharge amount of the sludge solution may be installed in the liquid phase outlet or the pipe connected thereto.

The sludge in the waste liquid falls vertically downward in the interior of the sludge separator due to its weight and accumulates on the bottom of the container, and the evaporated gas moves vertically upward due to its light density. However, the flow of the gas moving vertically upward obstructs the flow of the sludge falling downward, so that a part of the light-weight sludge moves together with the flow of the gas vertically upward. A means for preventing the inflow of sludge that moves together with the gas vertically upward in this way is provided on an upper side of the interior of the sludge separator.

FIG. 2 shows the flow of gas in a filter of a mesh net according to the prior art. While the gas passes through such a filter of a mesh net, it can prevent sludge inflow. But there is a problem in that the sludge with viscosity gradually sticks to the mesh net and clogs the holes, thereby hindering the flow of the gas passing through the mesh net.

A sludge barrier according to an embodiment of the present invention can solve the problem of a filter of a conventional mesh net by having a specially designed barrier unit appropriately arranged within the sludge separator.

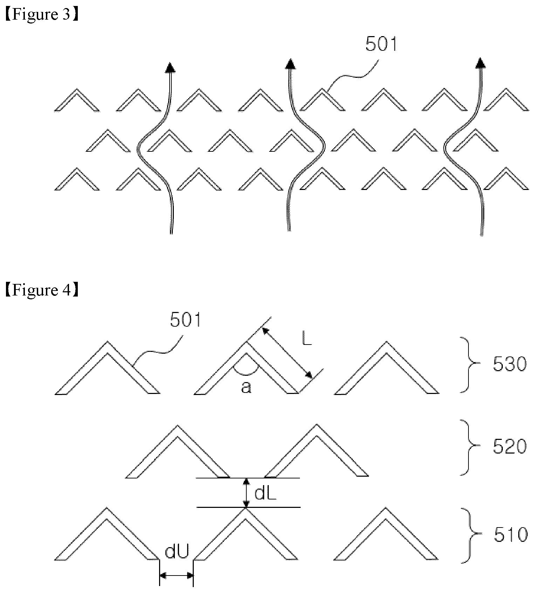

FIG. 3 shows the flow of gas in a sludge barrier according to an embodiment of the present invention.

Referring to FIG. 3, a sludge barrier (500) according to an embodiment has a plurality of barrier layers (510, 520, 530), and the barrier layer includes a plurality of barrier units (501) that have a cross-section of a conical hat shape and are spaced apart from each other.

In particular, the barrier unit (501) has a conical hat shape extended in one direction, and a plurality of barrier units (501) may be arranged in rows within an individual barrier layer.

The gas flowing in the vertical upward direction moves while being refracted along an empty space between barrier units of the conical hat shape, and in this process, the sludge that was moving with the gas is blocked by the barrier unit of the conical hat shape and falls again in the downward direction. In addition, even if the sludge is trapped and collected in the lower part of the conical hat, it does not block the space between barrier units, so it does not affect the flow of gas passing through the sludge barrier.

In the sludge barrier according to the embodiment, the barrier layer and the individual barrier units constituting the sludge barrier can exhibit more excellent performance by adjusting the specifications such as the spacing and size between them to a specific range.

FIG. 4 illustrates a cross-sectional view of a sludge barrier according to an embodiment of the present invention.

The sludge barrier may have a structure in which three or more barrier layers are stacked. Specifically, the sludge barrier may have a structure in which a first barrier layer (610), a second barrier layer (620), a third barrier layer (630), and the like are stacked.

Within the sludge barrier, the individual barrier layers (510, 520, 530) may be separated from each other by a constant vertical spacing. The vertical spacing between the barrier layers may be defined, for example, as the vertical spacing between the barrier units provided in mutually adjacent barrier layers.

In the sludge barrier, the sludge blockage performance can be further improved by adjusting the specifications such as the separation spacing (dL) between the barrier layers, the length (L) of one side of the barrier unit (501), and the separation spacing (dU) between the barrier units.

Referring to FIG. 4, the vertical spacing (dL) between the barrier layers (510, 520, 530) may be, for example, 25 mm or more, 30 mm or more, or 35 mm or more, and may also be 50 mm or less, 45 mm or less, or 40 mm or less. Specifically, the vertical spacing (dL) between the barrier layers (510, 520, 530) may be 25 mm to 50 mm. More specifically, the vertical spacing (dL) between the barrier layers may be 30 mm to 40 mm.

In addition, the vertical spacing (dL) between the barrier layers may be adjusted to be within a certain ratio range based on the height of the barrier layer. For example, the vertical spacing (dL) between the barrier layers may be a spacing corresponding to 50% to 80%, and more specifically 50% to 70%, based on the height of the barrier layer.

Referring to FIG. 4, the spacing (dU) between the barrier units (501) may be, for example, 35 mm or more, 40 mm or more, or 45 mm or more, and may also be 55 mm or less, 50 mm or less, or 45 mm or less. Specifically, the spacing (dU) between the barrier units (501) may be 35 mm to 55 mm. More specifically, the spacing (dU) between the barrier units (501) may be 40 mm to 50 mm.

In addition, the separation spacing (dU) between the barrier units (501) may be adjusted to be within a certain ratio range based on the horizontal size of the barrier unit. For example, the separation spacing (dU) between the barrier units (501) may be a spacing corresponding to 30% to 50%, and more specifically 35% to 45%, based on the horizontal size of the barrier unit.

Referring to FIG. 4, the length (L) of one side of the conical hat shape in the cross-section of the barrier unit (501) may be, for example, 40 mm or more, 50 mm or more, or 60 mm or more, and may also be 100 mm or less, 85 mm or less, or 75 mm or less. Specifically, the length (L) of one side of the conical hat shape in the cross-section of the barrier unit (501) may be 40 mm to 100 mm. More specifically, the length (L) of one side of the conical hat shape in the cross-section of the barrier unit (501) may be 50 mm to 75 mm.

For example, an angle (a) at which a cross-section of the barrier unit (610) is bent into a conical hat shape may be 60° to 120°. More specifically, the angle (a) at which the cross-section of the barrier unit (610) is bent into a conical hat shape may be 75° to 105°.

In particular, barrier units (501) provided in barrier layers adjacent to each other may be arranged to be staggered from each other.

Specifically, a center of a barrier unit provided in one barrier layer may be located on the same vertical line as an edge of a barrier unit provided in an adjacent barrier layer or a gap between the barrier units. Accordingly, an effect of smoothly passing gas while blocking sludge can be enhanced.

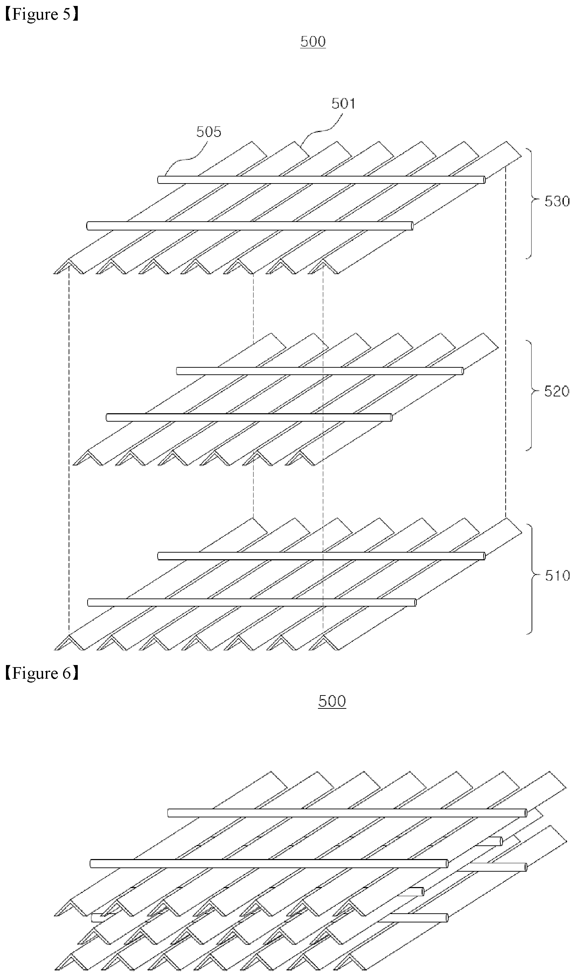

FIG. 5 illustrates an exploded perspective view of a sludge barrier according to an embodiment of the present invention. FIG. 6 illustrates a perspective view of a sludge barrier according to an embodiment of the present invention.

Referring to FIGS. 5 and 6, the sludge barrier (500) may have a structure in which three or more barrier layers (510, 520, and 530) are stacked.

The barrier unit (501) has a conical hat shape extending in one direction, and a plurality of barrier units may be arranged in rows within individual barrier layers (510, 520, and 530).

The plurality of barrier units (501) arranged in the rows may be fastened to a connection beam (505) that is arranged in a vertical direction with respect to the rows.

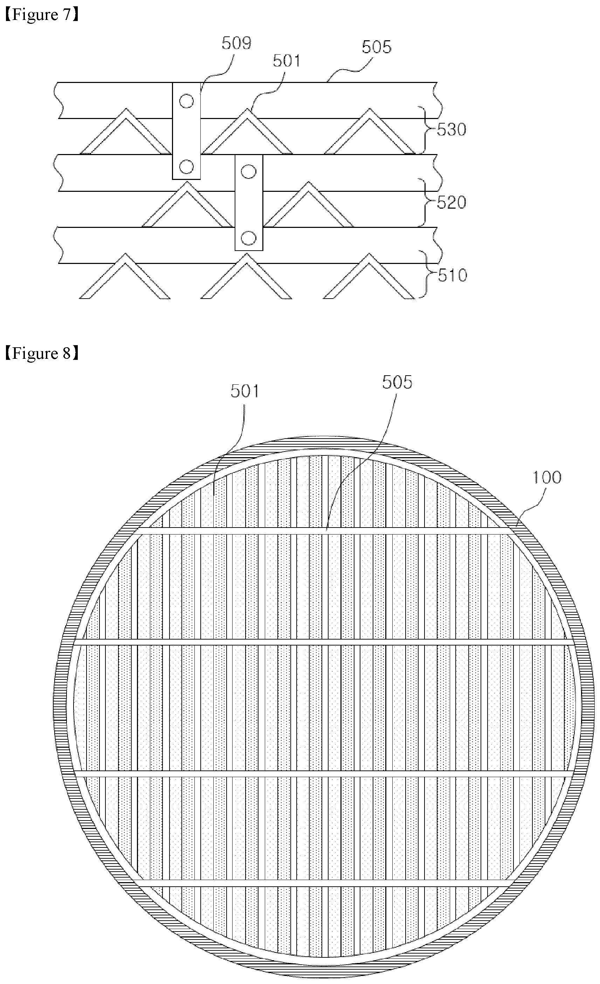

FIG. 7 illustrates a fastening structure of a barrier unit and connection beams according to an embodiment.

FIG. 8 illustrates a plan view of a sludge barrier according to an embodiment of the present invention.

Referring to FIG. 7, at least a part of the barrier unit (501) may be fastened in contact with the connection beam (505). For example, the apex portion of the conical hat shape of the barrier unit may penetrate and be fastened to the connection beam. More specifically, grooves are provided in the connection beam at constant spacing, and the apex portion of the conical hat shape of a barrier unit may be fastened in each of the grooves.

In addition, the connection beam may be fastened by a fastening means to another connection beam located above or below it, so that the barrier units and the connection beams constituting the sludge barrier can maintain an entirely coupled state. As an example, a connection beam fastened with a barrier unit provided in one barrier layer may be fastened by a fastening means with a connection beam fastened with a barrier unit provided in an adjacent barrier layer.

Specifically, a connection beam fastened with a barrier unit provided in the first barrier layer (510) may be fastened by a fastening means (509) with a connection beam fastened with a barrier unit provided in the second barrier layer (520). In addition, a connection beam fastened with a barrier unit provided in the second barrier layer (520) may be fastened by a fastening means (509) with a connection beam fastened with a barrier unit provided in the third barrier layer (530). Accordingly, the barrier units (501) and the connection beams (505) of all the barrier layers (510, 520, and 530) constituting the sludge barrier can all maintain a fastened state.

In addition, both ends of the connection beam (505) may be coupled to an inner wall of the container (100).

The sludge barrier may have a porosity within a certain range. If the porosity of the sludge barrier is too small, the flow of gas passing therethrough is not smooth, and if the porosity is too large, the performance of filtering sludge may not be sufficient.

For example, the porosity of the sludge barrier (500) may be 80% or more. Specifically, the porosity of the sludge barrier (500) may be 80% to 90%. More specifically, the porosity of the sludge barrier (500) may be 80% to 85%.

Such porosity can be calculated, for example, by calculating the ratio of the total pore area to the cross-sectional area of the sludge barrier.

The type of sludge blocked by the sludge barrier (500) may be, for example, suspended solids in the waste solution, a substance precipitated by a change in temperature, etc., but is not limited thereto.

The sludge separator may further include a separation plate (600) disposed in the container interior in the vicinity of the inlet (200).

As such, the separation plate may be provided at a position where the waste liquid is introduced into the sludge separator. At least a part of the separation plate may be attached to the inner wall of the container, and the separation plate may be provided at a position lower than the sludge barrier.

The separation plate can prevent the waste liquid injected into the container from directly contacting the sludge barrier (500). In addition, the separation plate can guide the initial flow direction of the waste liquid injected into the container.

The separation plate (600) may have, for example, a shape bent in the downward direction. Specifically, the separation plate may have a cross-section in an "L" shape or a diagonal shape, but is not limited thereto.

Referring to FIG. 1, when the cross-section of the separation plate (600) is an "L" shape, the inner diameter of the separation plate may be 500 mm to 800 mm, and the vertical size may be 300 mm to 500 mm. More specifically, the inner diameter of the separation plate (600) may be 600 mm to 700 mm, and the vertical size may be 350 mm to 450 mm. Here, the inner diameter of the separation plate means the horizontal size to the point bent in a downward direction, such as in an "L" shape.

Such a sludge separation plate can primarily filter the sludge by obstructing the flow of the waste liquid introduced into the sludge separator, thereby not only enabling the removal of the sludge with high efficiency but also extending the lifespan of the sludge barrier.

The sludge separator may be used for the purification of waste NMP.

Waste NMP recovered after being used as a material for a lithium secondary battery may contain a large amount of sludge. Waste NMP containing such a large amount of sludge is not easy to transfer in the process of recovering NMP and can block a transfer pipe, and a separate solution may be required for its transfer.

However, by using the sludge separator described above for the purification of waste NMP, it is possible to effectively separate the large amount of sludge contained in the waste NMP, and as a result, improve the recovery rate of NMP recovered from the waste NMP and provide NMP with high purity.

### NMP purification method

An NMP purification method according to another aspect of the present invention comprises: (1) a step of introducing waste NMP from a raw material tank into a sludge separator; (2) a step of discharging a first gas mixture containing NMP separated in the sludge separator through an upper end of the sludge separator, and discharging a mixture containing sludge through a lower end of the sludge separator; (3) a step of introducing the discharged first gas mixture containing NMP into a first distillation tower to perform a first purification; (4) a step of discharging an effluent containing water separated through the first purification to the outside the system through an upper end of the first distillation tower, and discharging first bottoms through a lower end of the first distillation tower; (5) a step of introducing the discharged first bottoms into a second distillation tower to perform a second purification; (6) a step of discharging purified NMP separated through the second purification to the outside the system through an upper end of the second distillation tower, and discharging second bottoms through a lower end of the second distillation tower; (7) a step of introducing the mixture containing sludge discharged in step (2) into a filtrate recovery apparatus and performing a heat treatment; (8) a step of discharging third bottoms separated through the heat treatment to the outside the system through a lower end of the filtrate recovery apparatus, and discharging a second gas mixture containing NMP through an upper end of the filtrate recovery apparatus; (9) a step of introducing the second bottoms discharged in step (6) and the second gas mixture containing NMP discharged in step (8) into an impurity tank and mixing them; and (10) a step of circulating the mixture in the impurity tank to the raw material tank.

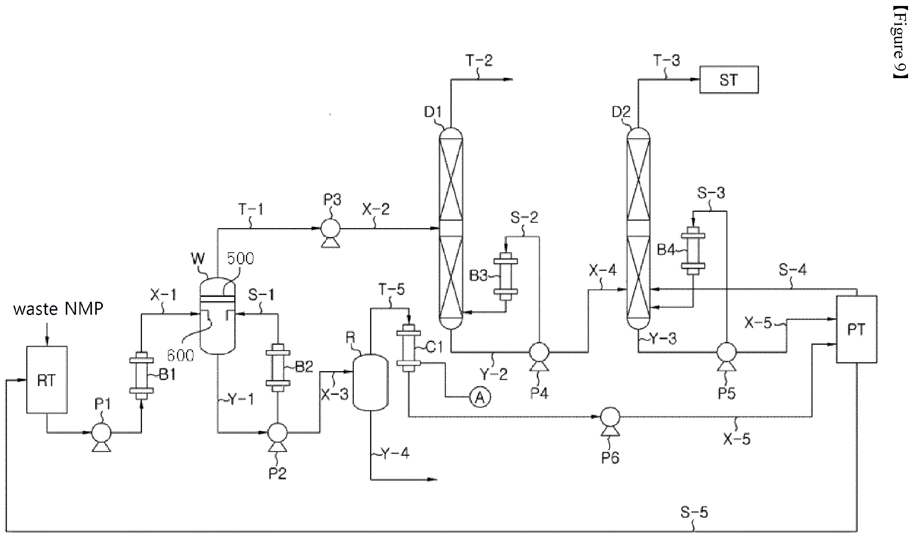

FIG. 9 illustrates an example of an NMP purification apparatus according to an embodiment of the present invention. Specifically, FIG. 9 illustrates an example of an NMP purification apparatus composed of a raw material tank (RT), a sludge separator (W), a first distillation column (D1), a second distillation column (D2), a filtrate recovery apparatus (R), and an impurity tank (PT).

Hereinafter, a method for purifying NMP according to an embodiment of the present invention will be described with reference to FIG. 9.

In step (1), waste NMP from the raw material tank is introduced into the sludge separator.

The NMP purification method according to an embodiment of the present invention includes a step of introducing waste NMP from the raw material tank into the sludge separator.

The waste NMP may be that which has been recovered after being used in a process of manufacturing a lithium secondary battery, but is not limited thereto. For example, the waste NMP may be that which has been recovered after being used as a material for a lithium secondary battery and may include a large amount of sludge.

The waste NMP may include NMP at 70 weight% or more. For example, the content of NMP in the waste NMP may be 72 weight% or more, 75 weight% or more, 78 weight% or more, 80 weight% or more, 85 weight% or more, 90 weight% or more, or 95 weight% or more, based on the total weight of the waste NMP.

In addition, the waste NMP may include moisture. For example, the moisture content in the waste NMP may be 30 weight% or less, 25 weight% or less, 20 weight% or less, or 15 weight% or less, based on the total weight of the waste NMP.

The detailed constitution and features of the sludge separator are as described above in the sludge separator according to an embodiment. The NMP purification method according to an embodiment of the present invention can effectively separate a large amount of sludge included in waste NMP by using the sludge separator having the technical characteristics, thereby providing excellent processability.

In addition, before introducing the waste NMP into the sludge separator in step (1), a step (1-1) of preheating the waste NMP to a temperature of 60°C to 100°C may be further included. For example, step (1-1) may be a step of preheating the waste NMP to a temperature of 65°C to 95°C, and the preheated waste NMP may be introduced into the sludge separator.

In step (2), a first gas mixture including NMP separated in the sludge separator is discharged through an upper end of the sludge separator, and a mixture including sludge is discharged through a lower end of the sludge separator.

The NMP purification method according to an embodiment of the present invention includes a step of discharging a first gas mixture including NMP separated in the sludge separator through an upper end of the sludge separator, and discharging a mixture including sludge through a lower end of the sludge separator.

Specifically, the step (2) can improve the processability of recovering NMP, and the recovery rate and purity of NMP by removing a large amount of sludge included in the waste NMP, by effectively separating the first gas mixture including NMP and the mixture including sludge separated using the sludge separator.

In step (3), the first gas mixture including the discharged NMP is introduced into the first distillation column to perform a first purification.

An NMP purification method according to an embodiment of the present invention includes a step of performing a first purification by introducing the first gas mixture containing the discharged NMP into the first distillation tower.

In the step (3), the first gas mixture containing the discharged NMP may be introduced into a middle-upper part of the first distillation tower. Specifically, it may be preferable for the first gas mixture containing the NMP to be introduced into an intermediate position or a higher upper part of the first distillation tower to perform the first purification more efficiently, but the present invention is not limited thereto.

The first purification of the step (3) may be performed at a temperature of 50°C to 100°C and a pressure of 100 torr to 130 torr. For example, the first purification may be performed at a temperature of 52°C to 85°C or 55°C to 75°C, and a pressure of 105 torr to 130 torr or 110 torr to 125 torr. When the temperature and the pressure condition of the first purification satisfy the range, the moisture contained in the first gas mixture containing NMP can be effectively removed.

In step (4), an effluent containing water separated through the first purification is discharged to the outside the system through the upper end of the first distillation tower, and first bottoms are discharged through the lower end of the first distillation tower.

An NMP purification method according to an embodiment of the present invention includes a step of discharging an effluent containing water separated through the first purification to the outside the system through the upper end of the first distillation tower, and discharging first bottoms through the lower end of the first distillation tower.

Specifically, the step (4) can improve the purity of NMP by lowering the content of moisture by separating an effluent containing water from the first gas mixture containing NMP through the first purification of the step (3) and discharging the effluent to the outside the system.

The moisture content in the first bottoms may be 0.01% or less. For example, the moisture content in the first bottoms may be 0.008% or less, 0.006% or less, or 0.005% or less.

In addition, the first bottoms may contain NMP in an amount of 99.9 weight% or more. For example, the content of NMP in the first bottoms may be 99.9 weight% or more, 99.92 weight% or more, or 99.94 weight% or more, based on the total weight of the first bottoms. The purity of NMP in the first bottoms can be further improved by effectively removing most of the moisture in the waste NMP through the first purification.

In step (5), the discharged first bottoms are introduced into a second distillation tower to perform a second purification.

An NMP purification method according to an embodiment of the present invention includes a step of introducing the discharged first bottoms into a second distillation tower to perform a second purification.

The purity of NMP can be improved through the second purification. Specifically, the waste NMP may include, along with NMP, a component having a boiling point higher than that of NMP, and by performing a second purification in step (5) on the first bottoms from which moisture has been removed through the first purification, the component having a boiling point higher than that of NMP can be effectively removed, so that the purity of NMP can be further improved.

In the step (5), the first bottoms may be introduced into a middle-lower part of the second distillation tower. Specifically, it may be preferable for the first bottoms to be introduced into an intermediate position or a lower part lower than the intermediate position of the second distillation tower to more efficiently perform the second distillation, but the present invention is not limited thereto.

The second purification may be performed at a temperature of 120°C to 150°C and a pressure of 50 torr to 90 torr. For example, the second purification may be performed at a temperature of 122°C to 148°C or 126°C to 145°C, and a pressure of 55 torr to 85 torr or 65 torr to 80 torr. As the temperature and pressure conditions of the second purification satisfy these ranges, a component having a higher boiling point than NMP included in the first bottoms can be effectively removed.

In step (6), the purified NMP separated through the second purification is discharged to outside the system through an upper end of the second distillation tower, and second bottoms are discharged through a lower end of the second distillation tower.

An NMP purification method according to an embodiment of the present invention includes a step of discharging the purified NMP separated through the second purification to outside the system through the upper end of the second distillation tower, and discharging the second bottoms through the lower end of the second distillation tower.

Specifically, in the step (6), purified NMP can be obtained by discharging the second bottoms including a component having a higher boiling point than NMP from the first bottoms through the second purification of step (5).

The second bottoms may include 1.0 weight% or less of a component having a higher boiling point than NMP. For example, the content of the component having a higher boiling point than NMP in the second bottoms may be 0.8 weight% or less, 0.6 weight% or less, or 0.4 weight% or less, on the basis of the total weight of the second bottoms.

The second bottoms may include 99 weight% or more of NMP. For example, the content of NMP in the second bottoms may be 99.2 weight% or more, 99.4 weight% or more, or 99.6 weight% or more, on the basis of the total weight of the second bottoms.

A purity of the purified NMP may be 99.9% or more. For example, the purity of the purified NMP may be 99.92% or more, 99.94% or more, or 99.95% or more.

In step (7), a mixture including the sludge discharged in step (2) is inputted into a filtrate recovery apparatus and subjected to heat treatment.

An NMP purification method according to an embodiment of the present invention includes a step of inputting the mixture including the sludge discharged in step (2) into a filtrate recovery apparatus and subjecting the mixture to heat treatment.

Specifically, since a mixture including the sludge separated through the sludge separator may also include NMP, NMP included in the mixture including the sludge can be separated in a gaseous form and recycled by inputting the mixture including the sludge into the filtrate recovery apparatus and subjecting the mixture to heat treatment.

In step (7), the heat treatment may be performed at a temperature of 110°C to 140°C and a pressure of 60 torr to 100 torr. For example, the heat treatment may be performed at a temperature of 115°C to 140°C, 125°C to 138°C, or 128°C to 135°C, and a pressure of 65 torr to 95 torr or 75 torr to 85 torr. As process conditions of the heat treatment satisfy these ranges, a recovery rate of NMP can be further improved.

In addition, the pressure of the filtrate recovery apparatus can be controlled through a vacuum pump connected to the filtrate recovery apparatus. Since it is important to control the pressure of the filtrate recovery apparatus to obtain the effect intended by the present invention, the pressure of the filtrate recovery apparatus can be controlled through the vacuum pump.

In step (8), the third bottoms separated through the heat treatment is discharged to the outside the system through the lower end of the filtrate recovery apparatus, and a second gas mixture including NMP is discharged through the upper end of the filtrate recovery apparatus.

An NMP purification method according to an embodiment of the present invention includes a step of discharging the third bottoms separated through the heat treatment to the outside the system through the lower end of the filtrate recovery apparatus, and discharging a second gas mixture including NMP through the upper end of the filtrate recovery apparatus.

Specifically, step (7) can separate the third bottoms and the second gas mixture including NMP through the heat treatment, and the second gas mixture including NMP can be circulated and used. Thus, processability and the recovery rate of NMP can be improved in step (8).

The second gas mixture including NMP may include a component having a higher boiling point than NMP in an amount of 30 weight% or less. For example, the content of the component having a higher boiling point than NMP in the second gas mixture including NMP may be 1.5 weight% or less, 1.0 weight% or less, 0.8 weight% or less, or 0.6 weight% or less based on the total weight of the second gas mixture including NMP.

In step (9), the second bottoms discharged in step (6) and the second gas mixture including NMP discharged in step (8) are introduced into an impurity tank and mixed.

An NMP purification method according to an embodiment of the present invention includes a step of introducing the second bottoms discharged in step (6) and the second gas mixture including NMP discharged in step (8) into an impurity tank and mixing them.

The mixture in the impurity tank may include a component having a higher boiling point than NMP in an amount of 1.5 weight% or less. For example, the content of the component having a higher boiling point than NMP in the mixture in the impurity tank may be 1.0 weight% or less, 0.8 weight% or less, 0.6 weight% or less, or 0.4 weight% or less based on the total weight of the mixture in the impurity tank.

According to another embodiment of the present invention, the method may further include step (9-1) of cooling the second gas mixture including NMP discharged in step (9) to 45°C or less before introducing it into the impurity tank.

Although the second gas mixture including NMP discharged in step (9) may be introduced into the impurity tank and circulated without separate treatment, step (9-1) of cooling to 40°C or less or 35°C or less using a cooler may be additionally performed before introducing it into the impurity tank in order to facilitate mixing with the second bottoms discharged in step (6).

In step (10), the mixture in the impurity tank is circulated to the raw material tank.

An NMP purification method according to an embodiment of the present invention includes a step of circulating the mixture in the impurity tank to the raw material tank.

Specifically, the recovery rate of NMP can be improved by introducing and circulating the mixture in the impurity tank into the raw material tank. In particular, through this circulation, the component having a higher boiling point than NMP acts as a transfer material for waste NMP, thereby facilitating its use in the NMP purification process and improving processability.

According to another embodiment of the present invention, the method may further include step (11) of heat-treating the mixture of the impurity tank and feeding it into the second distillation tower. Specifically, the recovery rate of NMP can be further improved since the mixture of the impurity tank can be fed into the raw material tank and circulated, and can be heat-treated and then fed into the second distillation tower and circulated.

In the step (11), the heat treatment may be performed at 100°C to 130°C. For example, in the step (11), the heat treatment may be performed at a temperature of 105°C to 130°C or 115°C to 130°C.

In addition, the mixture of the impurity tank heat-treated in the step (11) may be fed into a middle or lower part of the second distillation tower. Specifically, it may be preferable for the gas mixture to be fed into an intermediate position of the second distillation tower or a lower part lower than that to perform more effective purification, but the present invention is not limited thereto.

More specifically, the position at which the mixture of the impurity tank heat-treated in the step (11) is fed into the second distillation tower may be the same as or lower than the position at which the first bottoms in the step (5) is fed into the second distillation tower. As the position at which the mixture of the impurity tank heat-treated in the step (11) is fed is the same as or lower than the position at which the first bottoms in the step (5) is fed, NMP can be more effectively purified. In particular, since the final product, high-purity NMP, is discharged to outside the system through the upper end of the second distillation tower, the first bottoms and the heat-treated mixture of the impurity tank should be fed into a middle or lower part of the second distillation tower to increase the efficiency of NMP purification, and more efficient purification can be performed by setting the input position of the heat-treated mixture of the impurity tank, which has a low content of NMP, to be relatively lower than the input position of the first bottoms.

### NMP purification apparatus

An NMP purification apparatus according to another aspect of the present invention comprises: a raw material tank that supplies waste NMP; a sludge separator having the above-described constitution, which discharges a first gas mixture including NMP separated from the supplied waste NMP through an upper end and discharges a mixture including sludge through a lower end; a first distillation tower that performs a first purification on the first gas mixture including the NMP, discharges an effluent including water separated through the first purification to the outside the system through an upper end, and discharges first bottoms through a lower end; a second distillation tower that performs a second purification by distilling the first bottoms, discharges purified NMP separated through the second purification to the outside the system through an upper end, and discharges second bottoms through a lower end; a filtrate recovery apparatus that performs a heat treatment on the mixture including sludge discharged from the sludge separator, discharges third bottoms separated through the heat treatment to the outside the system through a lower end, and discharges a second gas mixture including NMP through an upper end; and an impurity tank that mixes the second bottoms discharged from the second distillation tower and the second gas mixture including NMP discharged from the filtrate recovery apparatus and feeds the mixture into the raw material tank.

FIG. 9 illustrates an example of an NMP purification apparatus according to another embodiment of the present invention. Specifically, FIG. 9 exemplifies an NMP purification apparatus comprising a raw material tank (RT), a sludge separator (W), a first distillation tower (D1), a second distillation tower (D2), a filtrate recovery apparatus (R), and an impurity tank (PT).

First, waste NMP is supplied to the sludge separator (W) through the raw material tank (RT) (X-1). The supply may be performed through a first pump (P1), and at this time, the waste NMP may be preheated through a preheater (B1).

The sludge separator (W) may include a sludge filter (W1) provided in an upper part of the interior and a sludge separation plate (W2) provided on a side surface of the upper part of the interior at a position lower than the sludge filter.

In the sludge separator (W), a first gas mixture including NMP separated from the waste NMP or the preheated waste NMP through the sludge filter (W1) and the sludge separation plate (W2) is discharged through an upper end of the sludge separator (T-1) and input into the first distillation tower (D1) through a third pump (P3) (X-2), and a mixture including sludge is discharged through a lower end of the sludge separator (Y-1). The discharged mixture including sludge may be transferred to the filtrate recovery apparatus (R) through a second pump (P2) (X-3) and may be circulated back to the sludge separator (W) (S-1). At this time, the mixture including sludge may be heated through a sludge separator heater (B2) before being input back into the sludge separator (W).

The first distillation tower (D1) distills the first gas mixture including the input NMP for a first purification, an effluent including water separated through the first purification is discharged outside the system through an upper end of the first distillation tower (D1) (T-2), and first bottoms are discharged through a lower end of the first distillation tower (D1) (Y-2). The discharged first bottoms may be transferred to a second distillation tower (D2) through a fourth pump (P4) (X-3) and may be circulated back to the first distillation tower (D1) (S-2). At this time, the first bottoms may be heated through a first reboiler (B3) before being input back into the first distillation tower (D1).

The second distillation tower (D2) distills the supplied first bottoms for a second purification, purified NMP separated through the second purification is discharged outside the system through an upper end of the second distillation tower (D2) (T-3), and second bottoms are discharged through a lower end of the second distillation tower (D2) (Y-3). The purified NMP discharged outside the system may be stored in a storage tank (ST). In addition, the discharged second bottoms may be transferred to an impurity tank (PT) through a fifth pump (P5) (X-4) and may be circulated back to the second distillation tower (D2) (S-3). At this time, the second bottoms may be heated through a second reboiler (B4) before being input back into the second distillation tower (D2).

The filtrate recovery apparatus (R) performs heat treatment on the input mixture including sludge, and at this time, the pressure may be controlled through a vacuum pump (A). Third bottoms separated through the heat treatment are discharged outside the system through a lower end of the filtrate recovery apparatus (Y-4), and a second gas mixture including NMP is discharged through an upper end of the filtrate recovery apparatus (T-5) and is input to the impurity tank (PT) through a sixth pump (P6) (X-5). At this time, the second gas mixture including the discharged NMP may be input to the impurity tank (PT) after being cooled through a cooler (C1).

The impurity tank (PT) stirs and mixes the second bottoms discharged from the second distillation tower (D2) and the second gas mixture including NMP discharged from the filtrate recovery apparatus (R), and then circulates the mixture to the raw material tank (RT) (S-5). At this time, the mixture in the impurity tank may be heat-treated and input to the second distillation tower (D2) by additionally providing a heat treatment apparatus (S-4).

The NMP purification method and apparatus according to the present invention can improve the recovery rate of NMP recovered from waste NMP and provide NMP with high purity. Specifically, the NMP purification method according to an embodiment of the present invention purifies a first gas mixture containing NMP separated through a sludge separator before performing NMP purification, thereby improving not only the processability but also the recovery rate and purity of NMP. In particular, since the sludge separator includes a sludge barrier, it can simply and effectively separate a large amount of sludge contained in waste NMP without specially controlling the process conditions.

Also, a component with a higher boiling point than NMP contained in the mixture containing the sludge can be effectively removed by inputting the mixture containing the sludge separated through the sludge separator into the filtrate recovery apparatus and performing heat treatment.

In addition, after a second gas mixture containing NMP discharged from the filtrate recovery apparatus and second bottoms discharged through the second purification are input into an impurity tank and mixed, and then re-input into a raw material tank for circulation, not only can the recovery rate and purity of NMP be improved, but also a component with a higher boiling point than the circulated NMP acts as a transfer material for the waste NMP, thereby facilitating its use in the NMP purification process and improving processability.

Furthermore, when the mixture in the impurity tank is heat-treated and input into a second distillation tower, the recovery rate of NMP can be further improved.

## Claims

1. A sludge separator, comprising:
a container (100);
an inlet (200) through which waste liquid is supplied to the container;
a gas outlet (300) formed at an upper end of the container;
a liquid phase outlet (400) formed at a lower end of the container; and
a sludge barrier (500) disposed at an upper side of the container interior and having a plurality of barrier layers;
wherein each of the barrier layers comprises a plurality of barrier units (501) having a cross-section of a conical hat shape and spaced apart from each other.

2. The sludge separator of claim 1, wherein the barrier unit (501) has a conical hat shape extending in one direction, and a plurality of the barrier units are arranged in rows within each individual barrier layer.

3. The sludge separator of claim 1, wherein a spacing between the barrier units (501) is from 35 mm to 55 mm.

4. The sludge separator of claim 1, wherein a length of one side of the conical hat shape in a cross-section of the barrier unit (501) is from 40 mm to 100 mm.

5. The sludge separator of claim 1, wherein the barrier units provided in the adjacent barrier layers are arranged to be staggered from each other.

6. The sludge separator of claim 5, wherein the sludge barrier (500) has a structure in which three or more barrier layers are stacked, and the individual barrier layers are spaced apart from each other at a constant vertical spacing.

7. The sludge separator of claim 6, wherein a vertical spacing between the barrier layers is from 25 mm to 50 mm.

8. The sludge separator of claim 1, wherein a porosity of the sludge barrier (500) is from 80% to 90%.

9. The sludge separator of claim 2, wherein the plurality of barrier units (501) arranged in rows are fastened to a connection beam (505) arranged in a vertical direction with respect to these rows, and both ends of the connection beam (505) are coupled to an inner wall of the container (100).

10. The sludge separator of claim 1, wherein the sludge separator further comprises a separation plate (600) disposed in a container interior in a vicinity of the inlet, and the separation plate (600) prevents a waste liquid injected into the container from directly contacting the sludge barrier (500).

11. The sludge separator of claim 10, wherein the separation plate (600) has a shape bent in a downward direction, an inner diameter of the separation plate is from 500 mm to 800 mm, and a vertical size is from 300 mm to 500 mm.

12. The sludge separator of claim 1, wherein the sludge separator is used for purification of waste NMP.

13. A method for purifying NMP, comprising:
(1) a step of inputting waste NMP from a raw material tank into the sludge separator of claim 1;
(2) a step of discharging a first gas mixture comprising NMP separated in the sludge separator through an upper end of the sludge separator, and discharging a mixture comprising sludge through a lower end of the sludge separator;
(3) a step of inputting the discharged first gas mixture comprising NMP into a first distillation tower to perform a first purification;
(4) a step of discharging an effluent comprising water separated through the first purification to outside the system through an upper end of the first distillation tower, and discharging first bottoms through a lower end of the first distillation tower;
(5) a step of inputting the discharged first bottoms into a second distillation tower to perform a second purification;
(6) a step of discharging purified NMP separated through the second purification to outside the system through an upper end of the second distillation tower, and discharging second bottoms through a lower end of the second distillation tower;
(7) a step of inputting the mixture comprising sludge discharged in step (2) into a filtrate recovery apparatus and performing a heat treatment;
(8) a step of discharging third bottoms separated through the heat treatment to outside the system through a lower end of the filtrate recovery apparatus, and discharging a second gas mixture comprising NMP through an upper end of the filtrate recovery apparatus;
(9) a step of inputting the second bottoms discharged in step (6) and the second gas mixture comprising NMP discharged in step (8) into an impurity tank and mixing them; and
(10) a step of circulating the mixture from the impurity tank to the raw material tank.

14. An NMP purification apparatus, comprising:
a raw material tank configured to supply waste NMP;
a sludge separator having the constitution of claim 1, and configured to discharge a first gas mixture comprising NMP separated from the supplied waste NMP through an upper end, and to discharge a mixture comprising sludge through a lower end;
a first distillation tower configured to first purify the first gas mixture comprising the NMP, to discharge an effluent comprising water separated through the first purification to outside the system through an upper end, and to discharge first bottoms through a lower end;
a second distillation tower configured to second purify by distilling the first bottoms, to discharge purified NMP separated through the second purification to outside the system through an upper end, and to discharge second bottoms through a lower end;
a filtrate recovery apparatus configured to perform a heat treatment on the mixture comprising the sludge discharged from the sludge separator, to discharge third bottoms separated through the heat treatment to outside the system through a lower end, and to discharge a second gas mixture comprising NMP through an upper end; and
an impurity tank configured to mix the second bottoms discharged from the second distillation tower and the second gas mixture comprising NMP discharged from the filtrate recovery apparatus, and to input a resulting mixture into the raw material tank.
